# EUROPÄISCHE PATENTSCHRIFT

(11) **EP 1 117 678 B1**
(45) Veröffentlichungstag und Bekanntmachung des Hinweises auf die Patenterteilung: **21.04.2004**
(21) Anmeldenummer: 99948888.5
(22) Anmeldetag: 29.09.1999
(51) Int. Cl.: C07K 1/04, B01J 19/00, C07B 61/00, C07H 21/00

(54) **FESTPHASENSYNTHESE, WOBEI DIE FESTPHASE AUS ZWEI UNTERSCHIELDICHEN SUBSTRATEN BESTEHT**
SOLID PHASE SYNTHESIS, WHEREBY THE SOLID PHASE CONSISTS OF TWO DIFFERENT SUBSTRATES
SYNTHESE SUR PHASE SOLIDE, LA PHASE SOLIDE COMPRENANT DEUX SUBSTRATS DIFFERENTS

(30) Priorität: 30.09.1998 DE 19844988
(43) Veröffentlichungstag der Anmeldung: 25.07.2001
(73) Patentinhaber: Molecular Machines & Industries GmbH, 69120 Heidelberg (DE)
(72) Erfinder: SEEGER, Stefan, D-93077 Bad Abbach a.d. Donau (DE)
(74) Vertreter: HOFFMANN - EITLE
(86) Internationale Anmeldenummer: PCT/EP1999/007203
(87) Internationale Veröffentlichungsnummer: WO 2000/018792

(56) Entgegenhaltungen:
- WO-A-92/21079
- WO-A-97/19749
- WO-A-98/41534

## Beschreibung

Die Erfindung betrifft die Synthese von Substanzen an einer Festphase, wobei die Festphase aus zwei unterschiedlichen Substraten besteht, ein hierfür geeignetes Reaktionssystem sowie ein Verfahren umfassend eine Festphasensynthese und ein analytisches Verfahren, bei dem sowohl die chemische Struktur als auch die biologischen Eigenschaften der synthetisierten Substanz analysiert werden.

Die Synthese von Molekülen an einer Festphase ist insbesondere dann vorteilhaft, wenn eine Vielzahl aufeinanderfolgender Reaktionsschritte zum Aufbau des Moleküls erforderlich ist, da die Festphase eine leichte Abtrennung der jeweils erhaltenen Zwischenprodukte von den Reaktanden wie auch eine Automatisierung der Synthese ermöglicht. Insbesondere ist die Festphasensynthese die Standardsynthese bei der Herstellung von Polypeptiden und Polynukleotiden. Um eine möglichst große Fläche bei gegebenem Reaktionsvolumen bereitzustellen, finden hierbei in der Regel poröse Festphasen Anwendung.

In den letzten Jahren ist die Bedeutung der Festphasensynthese vor allem durch ihre Anwendung bei der Herstellung chemischer Bibliotheken stark gestiegen. Solche chemischen Bibliotheken sind beispielsweise durch das sog. "Split-Mix"-Verfahren hergestellt worden, bei denen Mikrokugeln, sogenannte Beads, auf verschiedene Reaktionsgefäße aufgeteilt werden, nach dem ersten Syntheseschritt, z.B. dem Anfügen des ersten Substituenten, wieder vereinigt und für den zweiten variablen Substituenten wieder aufgeteilt werden. Durch Wiederholen dieses Vorgangs ist es so auf technisch einfache Weise möglich, Tausende oder Millionen verschiedener Moleküle zu erzeugen, wobei sich auf jeder Mikrokugel nur eine bestimmte Molekülspezies befindet. Zur Herstellung einer ausreichenden Substanzmenge wie auch für eine leichte Handhabe der Festphase werden hierbei in der Regel poröse Mikrokugeln als Trägermaterial verwendet. Zum biologischen Screening der synthetisierten Substanz wird dann in der Regel das sog. FACS (Fluorescence Assisted Cell Sorting)-Verfahren direkt an der Mikrokugel angewendet, welches jedoch nur bei bestimmten Rezeptor-Ligand-Paaren durchführbar ist. Alternativ kann die Substanz von der Mikrokugel abgelöst und in gelöster Form einem Assay unterzogen werden, wobei u.a. eine Vermischung verschiedener Substanzen auftritt, welches wiederum nachteilig ist (Felder, Chimia 48, S- 531 - 541, 1994).

Als Alternative zu dem "Split-Mix"-Verfahren sind insbesondere Mehrfachparallelsynthesen vorgeschlagen worden, bei denen pro Reaktionsgefäß bzw. fester Reaktionsfläche eine bestimmte Spezies synthetisiert wird. Obwohl die Anzahl der synthetisierbaren Spezies gegenüber der "Split-Mix"-Technik geringer ist, weist dieses Verfahren den Vorteil auf, daß die Identität des Syntheseproduktes aufgrund der festen Position stets nachprüfbar ist und eine größere Menge synthetisiert werden kann. Die Synthese verläuft in der Regel an einem Träger mit einer möglichst großen Oberfläche wie z.B. porösen Harzträgern. Für biologische Assays muß daher das Syntheseprodukt wiederum von dem Träger abgelöst werden, so daß die Nachteile bei dem Screening von flüssigen Proben wie z.B. durch intermolekulare Wechselwirkungen bedingte Artefakte bestehen bleiben. Wird stattdessen die Synthese an einem nicht porösen, planaren Träger durchgeführt, ist die synthetisierte Menge insbesondere bei miniaturisierten Oberflächen für viele chemische Analysen zu gering.

Vor diesem Hintergrund war es das erfindungsgemäße Ziel, eine einfache Festphasensynthese bereitzustellen, bei der sowohl die Synthese einer ausreichenden Menge an Substanz zur chemischen Identifizierung der erhaltenen Substanz möglich ist, als auch die biologische Aktivität der erhaltenen Substanz gegenüber den Verfahren des Standes der Technik empfindlicher detektiert werden kann.

Dieses Ziel wird erfindungsgemäß erreicht durch eine Festphasensynthese von Substanzen in einem eine flüssige Phase enthaltendem Reaktionsgefäß, dadurch gekennzeichnet, daß die Festphase zur Synthese ein in die flüssige Phase reversibel eingeführtes Substrat und ganz oder teilweise die Gefäßwand des Reaktionsgefäßes umfaßt.

Vorzugsweise ist der Teil der Gefäßwand, der erfindungsgemäß als Festphase zur Synthese dient, der Boden des Reaktionsgefäßes. Dieser Boden ist vorzugsweise nicht porös und besonders bevorzugt nicht porös und planar.

Das in der flüssigen Phase reversibel eingeführte Substrat ist bevorzugt ein oder mehrere Mikrokugeln, beispielsweise Mikrokugeln aus einem Harz, beispielsweise einem chemisch modifizierten Polystyrol-Grundharz. Diese Mikrokugeln ermöglichen zum einen eine leichte Handhabe des Substrates nach erfolgter Synthese, zum anderen weisen sie eine große Oberfläche auf, so daß bei gegebenem Volumen die Menge an synthetisierter Substanz sehr groß wird. Alternativ kann das in der flüssigen Phase reversibel eingeführte Substrat auch die Spitze einer Nadel, wie z.B. einer Plastiknadel, sein, wie sie bereits bei Mehrfachparallelsynthesen eingesetzt wurden (Geysen et al, Proc.Natl. Acad.Sci.U.S.A.81, 3998, 1984).

Es ist hierbei erfindungswesentlich, daß bei jedem Syntheseschritt das reversibel in die flüssige Phase eingeführte Substrat wie auch der als Festphase dienende Teil der Gefäßwand des Reaktionsgefässes derart chemisch modifiziert vorliegen, daß beide Bestandteile der Festphase die gleichen Moleküle immobilisieren können und vorzugsweise eine annähernd gleiche Reaktivität aufweisen. Eine Vielzahl von Harzen für die Festphasensynthese ist im Handel erhältlich (beispielsweise von Calbiochem-Novabiochem GmbH, Schwalbach), die als reversibel in die flüssige Phase einführbare Substrate erfindungsgemäß verwendet werden können. Beispiele schliessen das Merrifield-Harz (Chlormethyl-derivatisiertes Polystyrolharz), Hydroxyfunktionalisierte Harze, Amino-funktionalisierte Harze, Trityl-Harze, Arylsilyloxy-Harze, Carboxy-funktionalisierte Harze, Aldehyd-funktionalisierte Harze, Thiolfunktionalisierte Harze, und Carbonat-Harze ein, an die in Abhängigkeit der reaktiven Gruppe verschiedene Moleküle mit unterschiedlichen reaktiven Gruppen in einer dem Fachmann geläufigen Art und Weise direkt oder über einen Spacer gebunden werden können. Ist ein bestimmtes Harz als reversibel einführbares Substrat für die erfindungsgemäße Synthese ausgewählt, so muß sichergestellt werden, daß der als Festphase dienende Teil der Gefäßwand des Reaktionsgefässes ganz oder teilweise funktionelle Gruppen trägt, die die Immobilisierung der gleichen Moleküle wie am reversibel einführbaren Substrat ermöglichen. Vorzugsweise weisen das reversibel einführbare Substrat und der als Festphase dienende Teil der Gefäßwand des Reaktionsgefässes die gleichen funktionellen Gruppen auf. Geeignete Verfahren zur Funktionalierung der Festphase sind dem Fachmann geläufig. Beispielsweise lassen sich Oberflächen in der Regel durch eine Silanisierung oder durch die Langmuir-Blodgett-Technik entsprechend funktionalisieren. Ist der als Festphase dienende Teil der Gefäßwand des Reaktionsgefässes beispielsweise ein Glasboden und ist ein Aminofunktionalisiertes Harz als reversibel einführbares Substrat ausgewählt worden, so kann die Aminofunktion auf der Glasoberfläche durch Derivatisierung mit beispielsweise Aminopropyl-trimethoxysilan generiert werden. Ist andererseits der Boden des Reaktionsgefässes beispielsweise ein Plexiglasboden und ist ein Carboxy-funktionalisiertes Harz als reversibel einführbares Substrat ausgewählt worden, so kann die Carboxyfunktion auf der Plexiglasoberfläche durch einen Langmuir-Blodgett-Film aufgebracht werden, wie beispielsweise in DE C 4332003 offenbart.

Wird der Synthesebaustein für die Festphasensynthese zu einer derart präparierten Festphase gegeben, sollte sichergestellt sein, daß die Menge an diesem Synthesebaustein gegenüber den an der Festphase gebundenen funktionellen Gruppen im Überschuß zugegeben wird. Bei ausreichender Reaktionszeit wird so sichergestellt, daß selbst bei Reaktivitätsunterschieden zwischen den funktionellen Gruppen am reversibel in die flüssige Phase eingeführten Substrat und an der Gefäßwand des Reaktionsgefäßes beide Bestandteile der Festphase vollständig abreagieren. Somit kann erreicht werden, daß auf der Gefäßwand des Reaktionsgefäßes und in dem reversibel eingeführten Substrat das gleiche Endmolekül synthetisiert wird. Ferner ist darauf zu achten, daß zwischen den einzelnen Syntheseschritten ausreichend gespült wird. Hierbei wird vorzugsweise die obere Öffnung des Reaktionsgefäßes mit einem Sieb versehen, welches sicherstellt, daß das reversibel eingeführte Substrat in dem Reaktionsgefäß während des Waschvorgangs verbleibt. Wird die Seitenwand des Reaktionsgefässes als Festphase genutzt, können die Reaktionslösungen auch durch einen porösen Boden des Reaktionsgefässes abgesaugt werden.

Im Gegensatz zu den aus dem Stand der Technik bekannten Mehrfachparallelsynthesen wird erfindungsgemäß erreicht, daß die Substanz nicht nur an einem in dem Reaktionsgefäß fest verankerten porösen Träger synthetisiert wird, sondern an der Gefäßwand des Reaktionsgefäßes sowie an einem in die flüssige Phase reversibel eingeführten Substrat, welches aus dem Gefäß leicht entfernt werden kann.

Die Vorzüge des erfindungsgemäßen Syntheseverfahrens liegen insbesondere in der gegenüber dem Stand der Technik deutlich vereinfachten Analyse der physikalisch-chemischen wie auch biologischen Eigenschaften der synthetisierten Substanz.

Eine Vielzahl biologischer Assays ist ohne vorhergehende Reaktions- und Trennschritte direkt mit dem erfindungsgemäß hergestellten, an der Festphase vorliegenden Syntheseprodukt möglich. Das reversibel in die flüssige Phase eingeführte Substrat wird nach erfolgter Synthese aus dem Reaktionsgefäß in einen anderen Behälter überführt. Die in dem Reaktionsgefäß verbliebenen Syntheseprodukte beispielsweise am Boden des Reaktionsgefässes können dann direkt einem biologischen Assay unterworfen werden. Die Wahl des biologischen Assays ist nicht beschränkt und schließt alle Assays ein, mit denen biologische bzw. biochemische Eigenschaften analysiert werden können. Hierzu zählen insbesondere Chemolumineszenzverfahren, Oberflächenplasmonenresonanzverfahren, Fluoreszenzverfahren sowie Oberflächentechniken unter Ausnutzung akustischer Veränderungen (akustische Sensoren). Die.erfindungsgemäßen Vorzüge treten jedoch insbesondere bei biologischen Assays zu tage, bei denen die Wechselwirkungen der synthetisierten Substanz mit Rezeptoren direkt in einer Zellmembran oder Ligand-Rezeptor-Wechselwirkungen, die zu einem für herkömmliche Assays zu schwachen Meßsignal führen, zu bestimmen sind.

Wechselwirkungen der synthetisierten Substanz mit Rezeptoren direkt in einer Zellmembran können mit Zellassays analysiert werden, die eine mikroskopische Beobachtung der Zellen durch dünne transparente Substrate umfassen. In diesem Fall ist der Boden des Reaktionsgefässes so gewählt, daß er für den entsprechenden Zellassay geeignet ist. Der Boden des Reaktionsgefässes ist bevorzugt ein Glassubstrat, im Falle eines Fluoreszenzassays mit z.B. Calcium-Imaging ein Quarzglasboden. Neben der einfacheren Durchführbarkeit durch direkte Synthese am Boden des Reaktionsgefässes weist der nach erfindungsgemässer Synthese durchgeführte Zellassay auch deshalb eine höhere Empfindlichkeit auf, weil alle Moleküle auf der Oberfläche in einer einheitlichen Orientierung vorliegen bzw. die Orientierung durch die Synthese kontrollierbar ist.

Zudem ist die erfindungsgemässe Synthese dann besonders vorteilhaft, wenn Ligand-Wechselwirkungen zu analysieren sind, für deren Analyse herkömmliche Assays zu unempfindlich oder zu unspezifisch wären. Durch das drastisch verminderte Detektionsvolumen im Falle der Detektion an einer als dünner Oberflächenfilm vorliegenden Substanz sind Assays möglich, die um Größenordnungen empfindlicher als entsprechende Volumen-Verfahren sind und bis zur Einzelmoleküldetektion gehen. Ein solcher Assay ist in der deutschen Patentanmeldung 198 22 542.4 (offenlegungsschrift DE 197 36 736) beschrieben und ermöglicht neben der Bestimmung absoluter Konzentrationen die Bestimmung von Affinitätskonstanten und Bindungskinetiken, die häufig ein Maß für die Spezifität der Wechselwirkung darstellen. Auch in diesem Fall ist der Boden des Reaktionsgefässes bei der erfindungsgemässen Synthese vorzugsweise ein Quarzglasboden.

Insbesondere wenn der biologische Assay ein interessantes Ergebnis zeigt, kann in einem zweiten Schritt die chemische Identität der getesteten Verbindung durch Analyse der zuvor aus dem Reaktionsgefäß entfernten Festphase bestimmt werden. Hierbei kommen klassische chemische Analysetechniken wie GC/MS, TOF/MS, MALDI und NMR in Betracht, abhängig von der Art der synthetisierten Substanz lassen sich jedoch auch Mikrosequenzierungen durchführen.

In einer erfindungsgemäßen bevorzugten Ausführungsform ist das Reaktionsgefäß eine Vertiefung einer Mikrotiterplatte, beispielsweise eine 96er, eine 384er oder eine 1536er Mikrotiterplatte. Eine solche Mikrotiterplatte ermöglicht es, bei Zugabe verschiedener Reaktanden in jeder Vertiefung parallel bis zu 1536 verschiedene chemische Synthesen durchzuführen. Weiterhin kommen mikrostrukturierte Systeme, etwa Systeme auf Grundlage der Wafertechnik, als erfindungsgemäß verwendbares Reaktionsgefäß in Betracht.

Die Erfindung stellt zudem ein Reaktionssystem bereit, welches für die erfindungsgemäße Festphasensynthese geeignet ist. Das erfindungsgemäße Reaktionssystem umfaßt eine Mikrotiterplatte, die mit einer flüssigen Phase gefüllt ist, das dadurch gekennzeichnet ist, daß die Vertiefungen der Mikrotiterplatte weiterhin jeweils ein reversibel in die flüssige Phase eingeführtes Substrat umfassen, wobei jeweils das Substrat und die Gefäßwand der entsprechenden Vertiefung ganz oder teilweise funktionelle Gruppen zur Immobilisierung der gleichen Moleküle tragen.

### Ausführungsbeispiel:

400 mg Polystyrolkugeln (Durchmesser 200 µm), die 200 µmol Amingruppen tragen, werden in 5 ml Dimethylformamid (DMF) gelöst und in eine Vertiefung einer Mikrotiterplatte gegeben, deren Boden aus einer 170 µm dicken Glasplatte besteht, die nach bekannten Methoden (z.B. S. Seeger et al. in: Synthetic Microstructures in Biological Research, Hrsg.: J. Schnur, M. Peckerar, Plenum Publishing Corporation, S. 53-66 (1992)) mit einer dünnen Schicht Ethoxy-aminosilan versehen wurde. Nun werden 1 mmol Fmoc-Aminosäure, 1 mmol Dicyclohexylcarbodiimid (DCC), 1 mmol N-Hydroxybenzotriazol (HOBt) und 1 mmol Dimethylaminopyridin (DMAP) zugegeben und über Nacht bei Raumtemperatur inkubiert.

Nach Waschen mit DMF wird die Schutzgruppe Fmoc durch 30 minütige Inkubation mit 20% Piperidin in DMF bei Raumtemperatur abgespalten und ein neuer Reaktionszyklus zur Präparation eines Festphasen-gekoppelten Dipeptids beginnt. Der Zyklus wird sechsmal wiederholt, so daß ein Hexapeptid entsteht. Die Seitenketten-Schutzgruppen werden schließlich mit 25% Trifluoressigsäure in Dichlormethan (30 min, Raumtemperatur) entfernt und gewaschen.

Je nach Sequenz der eingesetzten Aminosäuren entstehen in den einzelnen Vertiefungen der Platte entsprechende Hexapeptide. Die Mikrokugeln werden nun entnommen und können chemischen Analysemethoden unterzogen werden. Die am Glasboden der Mikrotiterplatte gebundenen Hexapeptide können leicht auf Bindung von Proteinen untersucht werden, indem fluoreszenzmarkierte Zielproteinmoleküle zugegeben werden und der Boden mit einem Fluoreszenzscanner abgerastert wird.

## Patentansprüche

1. Festphasensynthese von Substanzen in einem eine flüssige Phase enthaltenden Reaktionsgefäß, **dadurch gekennzeichnet, daß** die Festphase zur Synthese ein in der flüssigen Phase reversibel eingeführtes Substrat und ganz oder teilweise die Gefäßwand des Reaktionsgefässes umfaßt.

2. Festphasensynthese gemäß Anspruch 1, **dadurch gekennzeichnet, daß** das Reaktionsgefäß einen nicht porösen Boden als Teil der Gefäßwand aufweist, der als Festphase zur Synthese dient.

3. Festphasensynthese gemäß einem der Ansprüche 1 und 2, **dadurch gekennzeichnet, daß** die Gefäßwand des Reaktionsgefäßes und das reversibel eingeführte Substrat ganz oder teilweise die gleichen funktionellen Gruppen tragen.

4. Festphasensynthese gemäß einem der Ansprüche 2 und 3, **dadurch gekennzeichnet, daß** der Boden ein Quarzglasboden ist.

5. Festphasensynthese gemäß einem der vorhergehenden Ansprüche, **dadurch gekennzeichnet, daß** das in der flüssigen Phase reversibel eingeführte Substrat ein oder mehrere Mikrokugeln ist.

6. Festphasensynthese gemäß einem der Ansprüche 1 bis 4, **dadurch gekennzeichnet, daß** das in der flüssigen Phase reversibel eingeführte Substrat ein Nadelkopf ist.

7. Festphasensynthese gemäß einem der vorhergehenden Ansprüche, **dadurch gekennzeichnet, daß** das Reaktionsgefäß eine Vertiefung einer Mikrotiterplatte ist.

8. Verfahren umfassend (a) eine Festphasensynthese gemäß einem der vorhergehenden Ansprüche und (b) ein analytisches Verfahren,
umfassend (b1) die Analyse der chemischen Struktur der synthetisierten Substanz durch Analyse der an dem in der flüssigen Phase reversibel eingeführten Substrat synthetisierten Substanz, und
(b2) die Analyse der biologischen Eigenschaften der synthetisierten Substanz durch Analyse der an der Gefäßwand des Reaktionsgefässes synthetisierten Substanz.

9. Reaktionssystem, umfassend eine Mikrotiterplatte, die mit einer flüssigen Phase gefüllt ist, **dadurch gekennzeichnet, daß** die Vertiefungen der Mikrotiterplatte weiterhin jeweils ein reversibel in die flüssige Phase eingeführtes Substrat umfassen, wobei jeweils das Substrat und die Gewäßwand der entsprechenden Vertiefung ganz oder teilweise funktionelle Gruppen zur Immobilisierung der jeweils gleichen Moleküle tragen.

## Claims

1. Solid-phase synthesis of substances in a reaction vessel containing a liquid phase, **characterised in that** the solid phase for synthesis comprises a substrate reversibly introduced in the liquid phase and completely or partly the vessel wall of the reaction vessel.

2. Solid-phase synthesis according to claim 1, **characterised in that** the reaction vessel has a non-porous base as part of the vessel wall which serves as solid phase for synthesis.

3. Solid-phase synthesis according to one of claims 1 and 2, **characterised in that** the vessel wall of the reaction vessel and the reversibly introduced substrate carry completely or partly the same functional groups.

4. Solid-phase synthesis according to one of claims 2 and 3, **characterised in that** the base is a quartz glass base.

5. Solid-phase synthesis according to one of the preceding claims, **characterised in that** the substrate reversibly introduced in the liquid phase is one or more microspheres.

6. Solid-phase synthesis according to one of claims 1 to 4, **characterised in that** the substrate reversibly introduced in the liquid phase is a pinhead.

7. Solid-phase synthesis according to one of the preceding claims, **characterised in that** the reaction vessel is a depression of a microtitre plate.

8. Process comprising (a) a solid-phase synthesis according to one of the preceding claims and (b) an analytical process,
comprising (b1) analysis of the chemical structure of the synthesised substance by analysis of the synthesised substance on the substrate reversibly introduced in the liquid phase, and
(b2) analysis of the biological properties of the synthesised substance by analysis of the synthesised substance on the vessel wall of the reaction vessel.

9. Reaction system comprising a microtitre plate which is filled with a liquid phase, **characterised in that** the depressions of the microtitre plate also comprise in each case a substrate reversibly introduced into the liquid phase, wherein in each case the substrate and the vessel wall of the corresponding depression carry, completely or partly, functional groups for immobilising the particular same molecules.

## Revendications

1. Synthèse sur phase solide, de substances, dans un récipient -de réaction contenant une phase liquide, **caractérisée en ce que** la phase solide, prévue pour la synthèse, comprend un substrat introduit de façon réversible dans la phase liquide et, en totalité ou en partie, la paroi du récipient de réaction.

2. Synthèse sur phase solide selon la revendication 1, **caractérisée en ce que** le récipient de réaction présente, en tant que partie de la paroi de récipient, un fond non poreux, servant de phase solide pour la synthèse.

3. Synthèse sur phase solide selon l'une des revendications 1 et 2, **caractérisée en ce que** la paroi de récipient de réaction et le substrat, introduit de façon réversible, portent en totalité ou en partie les mêmes groupes fonctionnels.

4. Synthèse sur phase solide selon l'une des revendications 2 et 3, **caractérisée en ce que** le fond est un fond en verre au quartz.

5. Synthèse sur phase solide selon l'une des revendications précédentes, **caractérisée en ce que** le substrat, introduit de façon réversible dans la phase liquide, est une ou plusieurs micro-billes.

6. Synthèse sur phase solide selon l'une des revendications 1 à 4, **caractérisée en ce que** le substrat, introduit de façon réversible dans la phase liquide, est une tête d'aiguille.

7. Synthèse sur phase solide selon l'une des revendications précédentes, **caractérisée en ce que** le récipient de réaction est une cavité d'une plaque de micro-titrage.

8. Procédé comprenant (a) une synthèse sur phase solide selon l'une revendications précédentes, et (b) un procédé analytique,
comprenant (b1) l'analyse de la structure chimique de la substance synthétisée, par analyse de la substance synthétisée sur le substrat introduit de façon réversible dans la phase liquide, et
(b2) l'analyse des propriétés biologiques de la substance synthétisée, par analyse de la substance synthétisée sur la paroi du récipient de réaction.

9. Système de réaction, comprenant une plaque de micro-titrage, remplie d'une phase liquide, **caractérisée en ce que** les cavités de la plaque de micro-titrage comprennent, en outre, chacune, un substrat introduit de façon réversible dans la phase liquide, sachant que, chaque fois, le substrat et la paroi de récipient de la cavité correspondante portent des groupes totalement ou partiellement fonctionnels, pour l'immobilisation, chaque fois, de la même molécule.
